# EUROPÄISCHE PATENTSCHRIFT

(11) **EP 1 404 239 B1**
(45) Veröffentlichungstag und Bekanntmachung des Hinweises auf die Patenterteilung: **14.02.2007**
(21) Anmeldenummer: 02762355.2
(22) Anmeldetag: 12.07.2002
(51) Int. Cl.: A61B 17/34, A61M 25/04

(54) **INJEKTIONSVORRICHTUNG ZUM INJIZIEREN VON KAPSELN**
INJECTION DEVICE FOR INJECTING CAPSULES
DISPOSITIF D'INJECTION POUR INJECTER DES CAPSULES

(30) Priorität: 12.07.2001 DK 200101094
(43) Veröffentlichungstag der Anmeldung: 07.04.2004
(73) Patentinhaber: Bavarian Nordic A/S, 3490 Kvistgaard (DK)
(72) Erfinder: LÖHR, Matthias, 69469 Weinheim (DE); GÜNZBURG, Walter, A-2340 Mödling (AT)
(74) Vertreter: Konnerth, Dieter Hans
(86) Internationale Anmeldenummer: PCT/EP2002/007803
(87) Internationale Veröffentlichungsnummer: WO 2003/005913

(56) Entgegenhaltungen:
- WO-A-01/23031
- DE-C- 19 734 385
- FR-A- 2 564 734
- FR-A- 2 756 493
- US-A- 5 395 319
- US-A- 5 470 318
- US-A- 5 667 514
- US-A- 6 162 203

## Beschreibung

Die Erfindung betrifft eine Injektionsvorrichtung, mit der Kapseln in einen Körper eingebracht werden können, sowie ein Verfahren zum Einbringen von eine Substanz enthaltenden Kapseln in einen Körper mittels einer Injektionsvorrichtung.

Zum Einbringen eines flüssigen Medikaments in den Körper eines Patienten werden beispielsweise Katheter-Nadel-Einrichtungen verwendet. So ist in der US 5,470,318 eine derartige Einrichtung beschrieben, bei der mittels einer Hohlnadel ein Zielgebiet im Körper punktiert wird und durch die Hohlnadel ein flexibler Schlauch eingeführt wird. Über diesen Schlauch wird anschließend ein flüssiges Medikament zugeführt. Medikamente, die in druckempfindlichen Kapseln enthalten sind, können mittels einer derartigen Injektionsvorrichtung nicht verabreicht werden, da beim Injizieren die druckempfindlichen Kapseln aufplatzen und damit zerstört werden könnten.

Dokument FR-A-2756493 offenbart eine Injections vorrichtung gemaß dem Oberbegriff des ersten Anspruchs.

Aufgabe der Erfindung ist es, eine eingangs genannte Injektionsvorrichtung zu schaffen, die auch zum Einbringen von Substanzen oder Zellen in Form von druckempfindlichen Kapseln geeignet ist, sowie ein derartiges Verfahren anzugeben.

Diese Aufgabe wird erfindungsgemäß durch eine Injektionsvorrichtung mit den Merkmalen des Anspruchs 1 gelöst. Mit der Hohlnadel und der darin in eine solche Stellung eingeschobenen Schneidnadel, in der die Schneidspitze am Vorderende der Hohlnadel hervorsteht, wird das Zielgebiet im Körper, z. B. in einem Tumor oder allgemein in einem lebenden Organismus, punktiert. Anschließend wird die Schneidnadel aus der ortsfest gehaltenen Hohlnadel zurückgezogen und durch den Katheter ersetzt, der in die Hohlnadel in etwa bis zu deren Vorderende eingeschoben wird. Der Katheter enthält an seinem distalen Ende einen Aufnahmeraum für die Substanz oder ein Medikament, die bzw. das in Form von Kapseln vorliegt. In den Katheter ist ein Mandrin einschiebbar und in einer vorgesehenen Position positionierbar, in der er zumindest mit seiner Stirnseite den Aufnahmeraum rückseitig begrenzt. Durch das Einschieben des Katheters mit dem daran festgelegten Mandrin in die Hohlnadel werden die Kapseln in denjenigen Bereich im Körper gebracht, in dem sie freigesetzt werden sollen. Da sie vom Katheter umhüllt sind, wird auf sie jedoch kein Druck ausgeübt. Anschließend wird zunächst die aus Hohlnadel und Katheter gebildete Einheit gegenüber dem noch ortsfest gehaltenen Mandrin zurückgezogen und damit die äußere Umhüllung des Aufnahmeraumes entfernt. Je nach Gestaltung des Mandrins werden schon durch das Zurückziehen der äußeren Hülle und/oder bei dem anschließenden gemeinsamen Zurückziehen des Mandrins mit der Hohlnadel und dem Katheter die Kapseln in dem von der Hohlnadel gebildeten Einstechkanal zurückgelassen. Folglich werden durch die erfindungsgemäße Injektionsvorrichtung die Kapseln nicht mit Druck in den Körper injiziert, sondern sie werden in dem Aufnahmeraum im Katheter geschützt in das Zielgebiet gebracht und nach dem Entfernen der Umhüllung in dem Einstechkanal im wesentlichen drucklos freigegeben. Wenn die Hohlnadel beim Herausziehen des Katheters im Zielgebiet gehalten wird, kann ein weiterer mit Kapseln geladener Katheter in die Hohlnadel eingeschoben werden, dessen Kapseln in derselben Weise im Körper freigegeben werden. Somit eignet sich die Injektionsvorrichtung für eine Mehrfachinjektion an derselben Stelle oder in einem benachbarten Bereich des Körpers.

Mit der Injektionsvorrichtung können statt einer Substanz oder eines Medikaments allgemein auch Zellen, Mikrosphären, Lipidkörper, Fullerene und dergleichen, die in Kapseln oder ähnlichen druckempfindlichen Trägern enthalten sind, in einen Körper eingebracht werden. Unter Körper werden menschliche oder tierische Körper oder Körperteile oder sonstige Körper verstanden, in die aufgrund ihrer Beschaffenheit die Injektionsvorrichtung schneidend eindringen kann, wie z. B. Pflanzen, tierische oder pflanzliche Produkte, insbesondere im Lebensmittelsektor, oder auch andere technische Produkte oder Gegenstände, in die Substanzen oder Wirkstoffe zum Ausführen von Reaktionen einzubringen sind. Die Kapseln oder Träger sind beispielsweise derart gebildet, daß die enthaltenen Substanzen oder Wirkstoffe im Körper langsam freigesetzt werden können.

Vorteilhafte Ausgestaltungen der Erfindung sind in den Unteransprüchen angegeben.

Der Katheter enthält an seinem Vorderende ein entfernbares Sieb, das beim Beladen des Katheters ein Spülen der aufgenommenen Kapseln mit einer Spüllösung, insbesondere einer physiologischen Salzlösung gestattet, die durch das Sieb abtropfen kann. Dieses Sieb muß entfernt werden, wenn die Kapseln aus dem Aufnahmeraum des Katheters in den Körper freigegeben werden sollen. Dafür kann vorgesehen sein, daß ein das Sieb enthaltender Vorderabschnitt des Katheters an einer Materialschwächung abtrennbar ist. Unmittelbar vor dem Einschieben des Katheters in die Hohlnadel wird der das Sieb tragende Vorderabschnitt abgebrochen. Eine alternative Gestaltung sieht vor, daß der Mandrin an seinem Vorderabschnitt eine sich entlang des Aufnahmeraums erstreckende Verlängerung mit einer Schneideinrichtung zum Abtrennen des im Körper verbleibenden Siebs aufweist. Falls erforderlich, kann das Sieb aus einem sich zersetzenden Stoff gebildet sein und insbesondere ist es biologisch abbaubar. Bei der Relativverschiebung des Katheters gegenüber dem Mandrin trennt die Schneideinrichtung das Sieb ab, das im Körper verbleibt. Die Verlängerung kann beispielsweise von zwei oder mehreren stiftartigen Vorsprüngen gebildet sein, die von der Stirnseite des Mandrins vorstehen und zwei oder mehrere Schneidzähne oder einen Schneidring tragen.

Vorzugsweise enthält der Mandrin in seinem Vorderabschnitt eine sich nach vorne öffnende Höhlung, die den Aufnahmeraum bildet, und ein als zirkulärer Schneidring gebildetes Vorderende. Der Aufnahmeraum wird somit nicht unmittelbar von dem Katheter gebildet, sondern von der die Höhlung umgebenden Wand des Mandrins. Um das selbsttätige Herausbewegen der Kapseln aus dem Mandrin zu unterstützen und zu verbessern, ist die Höhlung bzw. der Aufnahmeraum mit einem sich zur Öffnung hin erweiternden Querschnitt versehen.

Der Mandrin ist zweckmäßigerweise derart gebildet, daß er am Katheter in einer ersten Position, in der sein Vorderende zum Begrenzen des Aufnahmeraumes vom Vorderende des Katheters zurückgesetzt ist, und in einer zweiten Position, in der sein Vorderende deckungsgleich ist mit dem Vorderende des Katheters, in einfacher Weise positioniert werden kann. Um den Positioniervorgang zu erleichtern, kann der Mandrin zumindest eine Markierung aufweisen. Des weiteren kann zweckmäßigerweise ein Mandrinstopper zum wahlweisen Einstellen einer Begrenzung der Bewegung des Mandrins relativ zum Katheter vorgesehen sein.

Die Injektions vorrichtung kann in einem Verfahren gebzaucht werden. Dabei wird eine Hohlnadel der Injektionsvorrichtung mit ihrem Vorderende in einem Zielgebiet im Körper positioniert, anschließend wird in die Hohlnadel ein Katheter eingeschoben, der an seinem distalen Ende einen mit Kapseln befüllten Aufnahmeraum und einen den Aufnahmeraum begrenzenden Mandrin enthält. Daraufhin werden der Katheter und die Hohlnadel gegenüber dem ortsfest gehaltenen Mandrin gemeinsam zurückgezogen, wobei die Kapseln im Zielgebiet des Körpers im wesentlichen drucklos freigegeben werden, und anschließend wird der Mandrin aus dem Körper zurückgezogen.

Beim Zurückziehen des Katheters gegenüber dem Mandrin wird ein am Vorderende des Katheters angebrachtes und den Aufnahmeraum distal begrenzendes Sieb mittels des Mandrins vom Katheter abgetrennt und die Kapsein werden beim Zurückziehen des Katheters und der Hohlnadel oder alternativ beim Zurückziehen des Katheters, der Hohlnadel und des Mandrins drucklos freigegeben.

Zweckmäßigerweise werden beim Verfahren der Katheter und die Hohlnadel gegenüber dem ortsfest gehaltenen Mandrin im wesentlichen um die Länge des Aufnahmeraums gemeinsam zurückgezogen und nach dem Freigeben der Kapseln werden der Katheter und der Mandrin aus der Hohlnadel vollständig zurückgezogen und die Hohlnadel wird in das Zielgebiet für nochmals einzubringende Kapseln vorgeschoben. Dann kann ein weiterer mit Kapseln befüllter und einen Mandrin enthaltender Katheter in die Hohlnadel eingeschoben werden, woraufhin die Kapseln wiederum im Körper in der voranstehend beschriebenen Weise drucklos freigegeben werden.

Bei dem Verfahren ist von Vorteil, daß die Kapseln im wesentlichen drucklos, insbesondere mittels einer Spritze, in den Aufnahmeraum eingefüllt werden können.

Des weiteren wird die Aufgabe durch eine Injektionsvorrichtung nach einem der Ansprüche 1 bis 9 zum Einbringen von eine Substanz enthaltenden Kapseln in einen Körper gelöst.

Nachfolgend wird die Erfindung anhand von Ausführungsbeispielen der Injektionsvorrichtung unter Bezugnahme auf die Zeichnungen näher erläutert. Es zeigt:
- Fig. 1: in einer Längsschnittansicht eine Injektionsvorrichtung mit einer Hohlnadel und einer Schneidnadel;
- Fig. 2: in einer Längsschnittansicht einen Katheter der Injektionsvorrichtung und einen im Katheter aufgenommenen Mandrin;
- Fig. 3: in einer Längsschnittansicht den Katheter und den Mandrin in einer alternativen Ausführungsform;

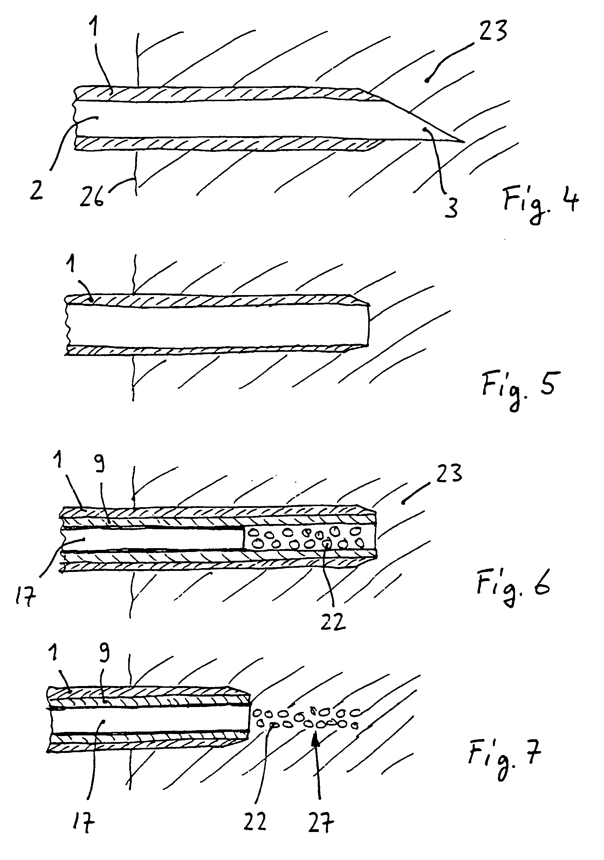

- Fig. 4: in einer Längsschnittansicht den Vorderabschnitt der Injektionsvorrichtung beim Einstechen in einen Körper;

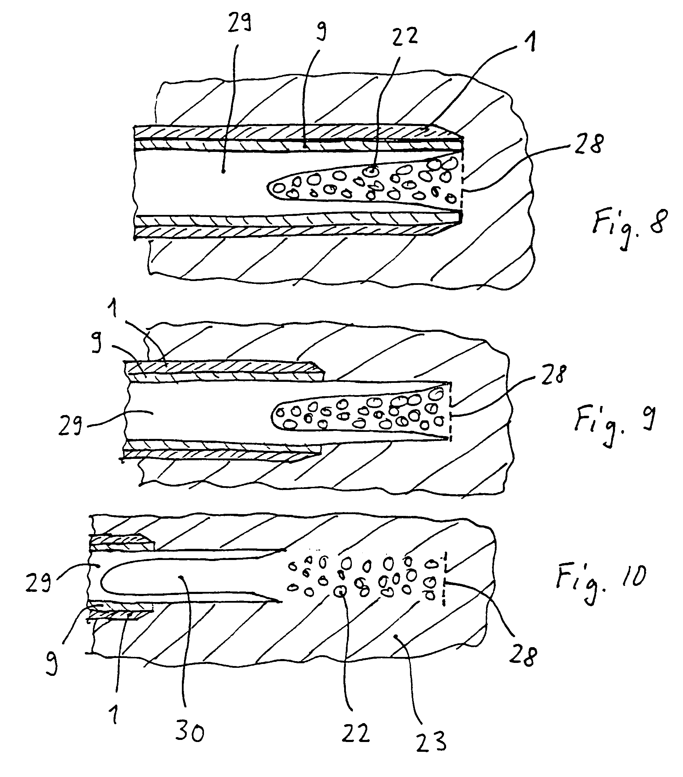

- Fig. 5: in einer Längsschnittansicht die im Körper positionierte Hohlnadel;
- Fig. 6: in einer Längsschnittansicht die Hohlnadel mit eingeschobenem und mit Kapseln befülltem Katheter;
- Fig. 7: in einer Längsschnittansicht die Injektionsvorrichtung beim Ausbringen der Kapseln;
- Fig. 8: in einer Längsschnittansicht die Hohlnadel mit der alternativ gestalteten Katheter-/Mandrin-Kombination;
- Fig. 9: in einer Längsschnittansicht die gegenüber dem Mandrin zurückgezogene Hohlnadel-/Katheter-Kombination; und
- Fig. 10: in einer Längsschnittansicht die Injektionsvorrichtung beim Zurückziehen aus dem Körper.

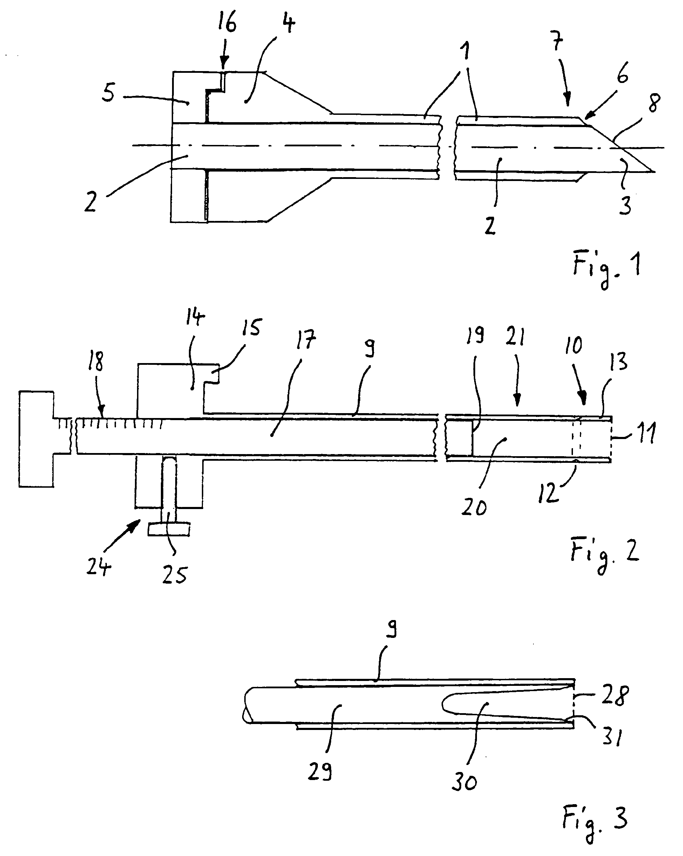

Eine Injektionsvorrichtung (siehe Fig. 1) enthält eine äußere Hohlnadel 1 und eine Schneidnadel 2, die spielfrei in der Hohlnadel 1 aufgenommen und darin verschiebbar ist. Die Schneidnadel 2 enthält eine atraumatisch angeschliffene vordere Schneidspitze 3. Die Hohlnadel 1 und die Schneidnadel 2 weisen jeweils an ihrem hinteren Ende ein gleichzeitig als Handhabe dienendes Befestigungsteil 4 bzw. 5 einer Verbindungs- oder Befestigungseinrichtung auf, z. B. einer Luer-Lock Verbindung zum lösbaren Festlegen der Schneidnadel 2 an der Hohlnadel 1, so daß die Schneidnadel 2 in der Hohlnadel 1 z. B. in der in Fig. 1 gezeigten Stellung festlegbar ist, in der die Schneidspitze 3 geringfügig, z. B. etwa 5 mm, am eine umlaufende Abschrägung 6 aufweisenden Vorderende 7 der Hohlnadel 1 hervorsteht, so daß die angeschliffene Abschrägung 8 der Schneidspitze 3 im wesentlichen stufenlos in die umlaufende Abschrägung 6 der Hohlnadel 1 übergeht.

Die Injektionsvorrichtung enthält des weiteren eine Beladeeinrichtung mit einem Katheter 9 (Fig. 2), der statt der Schneidnadel 2 in die Hohlnadel 1 spielfrei einführbar ist. Der Katheter 9 enthält an seinem Vorderende 10 ein Sieb 11 mit Sieböffnungen von etwa 0,1 mm Größe und eine vom Sieb 11 geringfügig zurückversetzte Sollbruchstelle 12, die beispielsweise in Form einer umlaufenden nutförmigen Materialschwächung oder Einkerbung gebildet ist, so daß das Sieb 11 durch Abbrechen des Siebträgerabschnitts 13 an der Sollbruchstelle 12 vom Katheter 9 entfernt werden kann. An seinem Hinterende enthält der Katheter 9 ein Befestigungsteil 14, mit dem durch Eingriff mit dem Befestigungsteil 4 der Hohlnadel 1 der Katheter 9 in der Hohlnadel 1 in Längsrichtung derart festgelegt werden kann, daß das an der Sollbruchstelle 12 endende Vorderende 10 des Katheters 9 mit dem Vorderende 7 der Hohlnadel 1 abschließt. Eine Festlegung in Umfangsrichtung kann mittels einer Nase 15 des Befestigungsteils 14 erfolgen, die in eine zugeordnete Ausnehmung 16 am Befestigungsteil 4 der Hohlnadel 1 eingreifen kann.

Der Katheter 9 ist z. B. aus Kunststoff hergestellt und kann eine Spitze aus einem mittels Röntgenstrahlung detektierbaren Material aufweisen. Die Länge des Katheters 9 beträgt z. B. 20 cm.

In dem Katheter 9 ist ein z. B. 30 cm langer Mandrin 17 (Fig. 2) spielfrei einsetzbar und darin verschiebbar. Der lange Mandrin 17 enthält an seinem Hinterabschnitt Markierungen 18 als Positionierhilfe beim Anordnen des Mandrins 17 in dem Katheter 9, so daß der Mandrin 17 auf einfache Weise in einer ersten Position, in der seine Stirnseite 19 deckungsgleich mit dem Vorderende 10 des Katheters 9 (bei abgebrochenem Sieb 11) angeordnet ist, und in einer zweiten Position, in der die Stirnseite 19 des Mandrins 17 z. B. um 5 cm zurückgezogen ist, so daß ein definierter Aufnahmeraum 20 im Vorderabschnitt 21 des Katheters 9 gebildet ist, positionierbar ist. Der Aufnahmeraum 20 ist beispielsweise für Kapseln 22 (Fig. 6), die ein Medikament enthalten und in einen Körper 23 eines Patienten eingebracht werden sollen, vorgesehen.

Mittels eines Mandrinstoppers 24 ist der Mandrin 17 relativ zum Katheter 9 festlegbar, so daß er entweder gänzlich unbeweglich am Katheter 9 oder zumindest gegen weiteres Einschieben in den Katheter 9 festgelegt ist. Der Mandrinstopper 24 enthält beispielsweise eine Stellschraube 25, die in dem den Mandrin 17 umgebenden, ringförmigen Befestigungsteil 14 des Katheters 9 radial verstellbar aufgenommen ist und den Mandrin 17 festklemmen kann. Der Mandrinstopper 24 kann auch als eigenständiger Ring gebildet sein, der auf dem Mandrin 17 verschiebbar und daran festlegbar ist und sich gegen das Hinterende des Katheters 9 abstützt, so daß das Einschieben des Mandrins 17 in den Katheter 9 begrenzt werden kann.

Die Injektionsvorrichtung wird in dem in Fig. 1 dargestellten Zustand, in dem die Schneidnadel 2 in der Hohlnadel 1 festgelegt ist, mit der Schneidspitze 3 durch die Haut 26 in ein Zielgebiet in einem Körper 23, z. B. in einen Tumor, eingeschoben und das Vorderende 7 der Hohlnadel 1 wird in der zum Einbringen der Kapseln 22 vorgesehenen Körperregion positioniert, beispielsweise mit Unterstützung einer Ultraschall- oder Röntgeneinrichtung, mittels der die aktuelle Position der Injektionsvorrichtung überwacht werden kann. Unter Beibehaltung der Position der Hohlnadel 1 wird die Schneidnadel 2 nach Lösen ihres Befestigungsteils 5 vom Befestigungsteil 4 der Hohlnadel 1 aus dieser zurückgezogen und der geladene Katheter 9, der in seinem Aufnahmeraum 20 die Kapseln 22 enthält und dessen das Sieb 11 enthaltendes Vorderende an der Sollbruchstelle 12 abgebrochen worden ist, wird in die Hohlnadel 1 eingeschoben (Fig. 6), bis die beiden Vorderenden deckungsgleich liegen, und mit dem Befestigungsteil 14 an der Hohlnadel 1 festgelegt. Ein kurzer Mandrin (z. B. 15 cm lang), der beim Beladen des Katheters 9 mit den Kapseln in den Katheter 9 vollständig eingeschoben wird und den Aufnahmeraum 20 begrenzt, wird gegen den langen Mandrin 17 ausgetauscht, der in seiner hinteren Position am Katheter 9 angeordnet wird und den befüllten Aufnahmeraum 20 rückseitig begrenzt.

Anschließend werden die Hohlnadel 1 und der Katheter 9, die miteinander über die Befestigungsteile 4 bzw. 5 verbunden sind, als Einheit bei geöffnetem Mandrinstopper 24 zurückgezogen und dabei relativ zum Mandrin 17, der in seiner bezüglich dem Körper ortsfesten Position gehalten wird, verschoben (Fig. 7). Die in dem Aufnahmeraum 20 enthaltenen Kapseln 22 werden somit beim Zurückziehen der zylindrischen Umhüllung aus dem Katheter 9 in dem im Körper gebildeten Einstechkanal 27 freigesetzt. Somit wird vermieden, daß Druck auf die Kapseln 22 ausgeübt wird, der ansonsten beim Einbringen der Kapseln 22 mittels einer Spritze erzeugt wird und die elastischen Kapseln 22 beschädigen könnte.

Wenn die Spitze des Katheters 9 bis zur Spitze des Mandrins 17 zurückgezogen ist (Fig. 7), sind die Kapseln 22 vollständig im Einstechkanal 27 im Gewebe des Körpers freigegeben und die Hohlnadel 1 mit dem Katheter 9 sowie der Mandrin 17 werden aus dem Körper 23 zurückgezogen.

Wenn anschließend ein weiteres Medikament in den Körper 23 an derselben Stelle eingebracht werden soll, wird, wie beschrieben, der Katheter 9 mit dem Mandrin 17 aus der Hohlnadel 1 zurückgezogen. Die Hohlnadel 1 verbleibt jedoch im Körper 23 und wird vor dem Einschieben des nächsten Katheters 9 in ihre ursprüngliche Position im Körper vorgeschoben. Anschließend wird ein mit Kapseln 22 befüllter, nächster Katheter 9 in die Hohlnadel 1 eingeführt und die Kapseln 22 werden in der beschriebenen Weise mittels der Injektionsvorrichtung in den Körper 23 eingebracht.

Zum Befüllen des Katheters 9 werden mittels einer Spritze Kapseln aus einem sterilen Behälter angesaugt. Die die Kapseln enthaltende Spritze wird anschließend an den Aufnahmeraum 20 des Katheters 9 angesetzt und die Kapseln werden durch langsames Vorschieben des Spritzenkolbens in den Katheter 9 ausgebracht. Der Katheter 9 wird dann senkrecht gehalten, so daß überschüssige Flüssigkeit durch das Sieb 11 abtropfen kann. Der Katheter 9 kann mittels einer sterilen Salzlösung gespült werden, bis die abtropfende Flüssigkeit rein ist. Anschließend wird der kurzer Mandrin in den Katheter 9 eingeführt und daran festgelegt, wobei der Aufnahmeraum 20 für die Kapseln in ausreichender Größe am Vorderabschnitt des Katheters 9 verbleibt. Der kurze Mandrin wird nach dem Einsetzen des Katheters 9 in die Hohlnadel 1 gegen den langen Mandrin 17 ausgetauscht.

In einer alternativen Ausführungsform enthält der Katheter 9 an seinem Vorderende ein Sieb 28 aus einem biologisch abbaubaren Material (Fig. 3). Der in den Katheter 9 einschiebbare Entlade-Mandrin 29 enthält eine zu seinem Vorderende hin geöffnete Höhlung als Aufnahmeraum 30 für die Kapseln 22 und einen zirkulären Schneidrand 31 mit einer zur Längsmittelachse geneigten Abschrägung. Im übrigen ist der Mandrin 29 entsprechend dem Mandrin 17 gebildet. Wenn der Mandrin 29 in den mit Kapseln 22 befüllten Katheter 9 eingeschoben wird, bewegen sich die Kapseln 22 in den Aufnahmeraum 30, während der Mandrin 29 mit seinem Schneidrand 31 bis unmittelbar an das Sieb 28 vorgeschoben und in dieser Stellung festgelegt wird (Fig. 8).

Zum Einbringen der Kapseln 22 wird entsprechend der vorangehenden Beschreibung der befüllte Katheter 9 in die im Körper 23 plazierte Hohlnadel 1 eingeschoben, bis die Vorderenden des Katheters 9 und der Hohlnadel 1 deckungsgleich liegen (Fig. 8). Beim Zurückziehen der Hohlnadel 1 mit dem daran festgelegten Katheter 9 trennt der ortsfest gehaltene Mandrin 29 mit seinem vorderen Schneidrand 31 das Sieb 28 vom Katheter 9 ab (Fig. 9), so daß der Aufnahmeraum 30 nach vorne hin geöffnet ist und die Kapseln 22 in den Einstechkanal 27 herausrollen können, wenn der Mandrin 29 ebenfalls zurückgezogen wird. Der Aufnahmeraum 30 kann einen sich von der Öffnung ins Innere verengenden Querschnitt, z. B. im Längsquerschnitt der Form einer Ellipse angenähert, aufweisen, so daß die Kapseln 22 unbehindert aus dem Aufnahmeraum 30 hinaus gelangen können. Das Sieb 28 verbleibt im Körper 23 und wird darin biologisch abgebaut.

### Bezugszeichenliste

- 1: Hohlnadel
- 2: Schneidnadel
- 3: Schneidspitze
- 4: Befestigungsteil
- 5: Befestigungsteil
- 6: Abschrägung
- 7: Vorderende
- 8: Abschrägung
- 9: Katheter
- 10: Vorderende
- 11: Sieb
- 12: Sollbruchstelle
- 13: Siebträgerabschnitt
- 14: Befestigungsteil
- 15: Nase
- 16: Ausnehmung
- 17: Mandrin
- 18: Markierung
- 19: Stirnseite
- 20: Aufnahmeraum
- 21: Vorderabschnitt
- 22: Kapsel
- 23: Körper
- 24: Mandrinstopper
- 25: Stellschraube
- 26: Haut
- 27: Einstechkanal
- 28: Sieb
- 29: Entlade-Mandrin
- 30: Aufnahmeraum
- 31: Schneidrand

## Patentansprüche

1. Injektionsvorrichtung, enthaltend
eine Hohlnadel (1),
eine in die Hohlnadel (1) einschiebbare Schneidnadel (2) mit einer am Vorderende (7) der Hohlnadel (1) vorstehenden Schneidspitze (3),
einen im Austausch gegen die Schneidnadel (2) in die Hohlnadel (1) in etwa bis zu deren Vorderende einschiebbaren Katheter (9), der an seinem distalen Ende einen Aufnahmeraum (20; 30) für eine in Kapselform vorliegende Substanz enthält, und
einen Mandrin (17; 29), der in den Katheter (9) einschiebbar und in einer den mit Kapseln (22) befüllten Aufnahmeraum (20: 30) am distalen Ende des Katheters (9) definierenden Stellung positionierbar ist,
wobei nach Anordnung der Injektionsvorrichtung in einem Zielgebiet eines Körpers (23) durch gemeinsame relative Rückzugsbewegung des Katheters (9) und der Hohlnadel (1) gegenüber dem Mandrin (17; 29) und anschließendes Zurückziehen des Mandrins (17; 29) die im Aufnahmeraum (20; 30) des eingeschobenen Katheters (9) enthaltenen Kapseln (22) im Zielgebiet des Körpers (23) im wesentlichen drucklos freigegeben werden,
**dadurch gekennzeichnet,**
**daß** der Katheter (9) an seinem Vorderende (10) ein entfernbares Sieb (11; 28) aufweist.

2. Injektionsvorrichtung nach Anspruch 1,
**dadurch gekennzeichnet, daß** ein das Sieb (11) enthaltender Vorderabschnitt (13) des Katheters (9) an einer Materialschwächung (12) abtrennbar ist.

3. Injektionsvorrichtung nach Anspruch 1 oder 2,
**dadurch gekennzeichnet daß** der Mandrin (29) an seinem Vorderabschnitt eine sich entlang des Aufnahmeraums (30) erstreckende Verlängerung mit einer Schneideinrichtung (31) zum Abtrennen des im Körper (23) verbleibenden und Siebs (28), das insbesondere biologisch abbaubar ist, aufweist.

4. Injektionsvorrichtung nach Anspruch 3,
**dadurch gekennzeichnet, daß** eine sich nach vorne öffnende Höhlung im Vorderabschnitt des Mandrins (29) den Aufnahmeraum (30) bildet und daß das Vorderende des Mandrins (29) als zirkulärer Schneidring (31) gebildet ist.

5. Injektionsvorrichtung nach Anspruch 4,
**dadurch gekennzeichnet, daß** sich die Höhlung bzw. der Aufnahmeraum (30) zur Öffnung hin erweitert.

6. Injektionsvorrichtung nach einem der Ansprüche 1 bis 5,
**dadurch gekennzeichnet, daß** der Mandrin (17; 29) am Katheter (9) in einer ersten Position, in der sein Vorderende zum Begrenzen des Aufnahmeraumes (20; 30) vom Vorderende des Katheters (9) zurückgesetzt ist, und in einer zweiten Position, in der sein Vorderende deckungsgleich ist mit dem Vorderende des Katheters (9), positionierbar ist.

7. Injektionsvorrichtung nach einem der Ansprüche 1 bis 6,
**dadurch gekennzeichnet, daß** der Mandrin (17; 29) zumindest eine Markierung (18) als Positionierhilfe aufweist.

8. Injektionsvonichtung nach einem der Ansprüche 1 bis 7,
**dadurch gekennzeichnet, daß** ein Mandrinstopper (24) zum wahlweisen Einstellen einer Begrenzung der Bewegung des Mandrins (17: 29) relativ zum Katheter (9) vorgesehen ist.

9. Injektionsvorrichtung nach einem der Ansprüche 1 bis 8,
**dadurch gekennzeichnet, daß** Katheter (9) einen röntgendichten Abschnitt oder eine röntgendichte Markierung enthält.

## Claims

1. Injection device, containing
a hollow needle (1),
a cutting needle (2) which can be inserted into the hollow needle (1) with a cutting tip (3) protruding from the front end (7) of the hollow needle (1),
a catheter (9) which can be inserted into the hollow needle (1) approximately as far as the front end of the hollow needle (1) as a replacement for the cutting needle (2), and having at its distal end a reservoir (20; 30) for a substance in the form of capsules, and
a mandrin (17; 29) which can be inserted into the catheter (9) and which can be positioned in the latter in a position defining the reservoir (20; 30) filled with capsules (22) at the distal end of the catheter (9),
the capsules (22) contained in the reservoir (20; 30) of the inserted catheter (9) being released largely without exerting any pressure in a target area of the body (23) after the injection device has been arranged in the target area of the body (23), by means of a common relative withdrawal movement of the catheter (9) and the hollow needle (1) in relation to the mandrin (17; 29) and subsequent withdrawal of the mandrin (17; 29),
**characterized in that**
the catheter (9) contains a removable sieve (11; 28) at its front end (10).

2. Injection device according to Claim 1, **characterized in that** a front end section (13) of the catheter (9) containing the sieve (11) can be separated off at a material weakening (12).

3. Injection device according to Claim 1 or 2, **characterized in that** the mandrin (29) has in its front section an extension extending along the reservoir (30) and having a cutting device (31) for separating off the sieve (28), which remains in the body (23) and which in particular is biodegradable.

4. Injection device according to Claim 3, **characterized in that**, in the front section of the mandrin (29), a cavity which is open towards the front forms the reservoir (30), and that the front end of the mandrin (29) is formed as a circular cutting ring (31).

5. Injection device according to Claim 4, **characterized in that** the cavity or reservoir (30) widens towards the opening.

6. Injection device according to one of Claims 1 to 5, **characterized in that** the mandrin (17; 29) can be positioned on the catheter (9) in a first position, in which its front end is set back from the front end of the catheter (9) in order to delimit the reservoir (20; 30), and in a second position, in which its front end coincides with the front end of the catheter (9).

7. Injection device according to one of Claims 1 to 6, **characterized in that** the mandrin (17; 29) has at least one mark (18) as a positioning aid.

8. Injection, device according to one of Claims 1 to 7, **characterized in that** a mandrin stopper (24) is provided for optionally setting a limit on the movement of the mandrin (17; 29) relative to the catheter (9).

9. Injection device according to one of Claims 1 to 8, **characterized in that** the catheter (9) contains a radiologically dense section or a radiologically dense mark.

## Revendications

1. Dispositif d'injection, comprenant
une aiguille creuse (1) ;
une aiguille tranchante (2) pouvant être insérée dans l'aiguille creuse (1) avec une pointe tranchante (3) prévue à l'extrémité avant (7) de l'aiguille creuse (1), un cathéter (9) pouvant être inséré en échange de l'aiguille tranchante (2) dans l'aiguille creuse (1) jusqu'à son extrémité avant environ, lequel cathéter comprend à son extrémité distale une chambre de réception (20 ; 30) pour une substance présente sous forme de capsule, et
un mandrin (17 ; 29), qui peut être inséré dans le cathéter (9) et qui peut être positionné à un emplacement défini de la chambre de réception (20 ; 30) remplie de capsules (22) à l'extrémité distale du cathéter (9),
moyennant quoi après la mise en place du dispositif d'injection dans une région cible d'un corps (23), par déplacement rotatif commun en arrière du cathéter (9) et de l'aiguille creuse (1) par rapport au mandrin (17; 29) et retrait par la suite du mandrin (17 ; 29), les capsules (22) contenues dans la chambre de réception (20; 30) du cathéter inséré (9) sont libérées essentiellement sans pression dans la région cible du corps (23),
**caractérisé en ce que**
le cathéter (9) présente un tamis amovible (11 ; 28) à son extrémité avant (10).

2. Dispositif d'injection selon la revendication 1,
**caractérisé en ce qu'**une section avant (13) contenant le tamis (11) du cathéter (9) peut être séparée au niveau d'un affaiblissement du matériau.

3. Dispositif d'injection selon la revendication 1 ou 2,
**caractérisé en ce que** le mandrin (29) présente, au niveau de sa section avant, une prolongation s'étendant le long de la chambre de réception (30) avec une installation de coupe (31) destinée détacher le tamis (28) restant dans le corps (23), lequel tamis est en particulier biodégradable.

4. Dispositif d'injection selon la revendication 3,
**caractérisé en ce qu'**un évidement s'ouvrant vers l'avant forme la chambre de réception (30) dans la section avant du mandrin (29) et **en ce que** l'extrémité avant du mandrin (29) a la forme d'une bague coupante circulaire (31).

5. Dispositif d'injection selon la revendication 4,
**caractérisé en ce que** l'évidement ou la chambre de réception (30) s'élargit vers l'ouverture.

6. Dispositif d'injection selon l'une quelconque des revendications 1 à 5,
**caractérisé en ce que** le mandrin (17 ; 29) est positionnable par rapport au cathéter (9) dans une première position, dans laquelle son extrémité avant est ramenée en arrière pour limiter la chambre de réception (20; 30) de l'extrémité avant du cathéter (9) et dans une seconde position, dans laquelle son extrémité avant coïncide avec l'extrémité avant du cathéter (9).

7. Dispositif d'injection selon l'une quelconque des revendications 1 à 6,
**caractérisé en ce que** le mandrin (17 ; 29) présente au moins un marquage (18) comme aide au positionnement.

8. Dispositif d'injection selon l'une quelconque des revendications 1 à 7,
**caractérisé en ce qu'**une butée de mandrin (24) est prévue pour régler de manière sélective une limitation du déplacement du mandrin (17 ; 29) par rapport au cathéter (9).

9. Dispositif d'injection selon l'une quelconque des revendications 1 à 8,
**caractérisé en ce que** le cathéter (9) comprend une section radio-opaque ou un marquage radio-opaque.
